# EUROPEAN PATENT SPECIFICATION

(11) **EP 1 803 389 B1**
(45) Date of publication and mention of the grant of the patent: **19.01.2011**
(21) Application number: 05425927.0
(22) Date of filing: 28.12.2005
(51) Int. Cl.: A61B 1/31, A61M 25/01

(54) **Self-propelled endoscopic device**
Selbstfortbewegende endoskopische Vorrichtung
Dispositif d'endoscope automoteur

(43) Date of publication of application: 04.07.2007
(73) Proprietor: Era Endoscopy S.r.l., 56025 Pontedera (Pisa) (IT)
(72) Inventor: Arena, Alberta, 56021 Marciana di Cascina Cascina (Pisa) (IT); Dario, Paolo, 57100 Livorno (IT); Gorini, Samuele, 56020 Montecalvoli (Pisa) (IT); Menciassi, Arianna, 56020 Pontedera (Pisa) (IT); Pernorio, Giuseppe, 56100 Pisa (IT)
(74) Representative: Bardini, Marco Luigi

(56) References cited:
- WO-A-02/068035
- US-A- 5 906 591
- US-A1- 2004 143 159

## Description

The present invention relates to an improvement to an endoscopic device for locomotion through a tubular body cavity and particularly, but not exclusively, through the gastrointestinal tract, capable of migrating in a preestablished direction by so-called inchworm motion.

More specifically the invention relates to an improvement to the system of anchoring an endoscopic device of the aforesaid type to the body cavity wall with the main aim of improving the locomotion efficiency.

Endoscopic devices for surgical or diagnostic procedures are already known. These devices are designed to be operated by the surgeon who directly imparts to the device its forward motion through the patient's body. These devices are generally associated with surgical and/or diagnostic instruments needed on each occasion to complete various procedures, e.g. micro-arms, micro-cameras and/or laser emitters.

Endoscopic devices of this type, but capable of autonomous or semi-autonomous locomotion through the body cavity of a patient, are described, for instance, in US-A-5398670, US-A-5906591 and WO02/068035. The endoscopic device described in these documents substantially consists of a tubular body of variable length with two end portions, said front and rear end portions, comprising anchoring means that enable the front end portion and the rear end portion to become temporarily and alternately attached to the wall of the body cavity, for example the intestine, to enable the forward motion of the device.

In particular, the variable-length tubular body of the endoscopic device described in the above-mentioned documents is in the form of a bellows-shaped tubular body and is consequently capable of extending and contracting as a result of admission and respectively aspiration of air therein. More specifically, during the extension phase, the bellows are pressurised by means of compressed air, obtaining an elongation proportional to the pressure injected, while the bellows are contracted by progressively reducing the pressure inside, until some degree of vacuum is created.

In the above-mentioned patent application PCT no. WO02/068035, the device is anchored to the wall of the body cavity by clamp means associated with the front and rear end portions of the device and selectively enabled by an external control unit in synchronism with the successive extensions and contractions of the bellows-shaped tubular body. The aforementioned clamp means are enabled by pneumatic actuating means which, in the preferred embodiment described in the aforesaid document, also consist of bellows-shaped members.

More particularly the clamp means comprise a pneumatically actuated mobile member and a fixed member. The mobile member, moving towards the fixed one, traps the tissue bordering the body cavity, achieving the grip necessary for locomotion. In order to allow the tissue to be drawn between the two members, a localised vacuum is generated between the latter which causes the surrounding tissue to collapse between them. This vacuum is generated by aspiration through a hole formed between the two gripping members and communicating with an external aspiration system.

A first problem found in the anchorage system provided in the endoscopic device according to patent application no. WO/02/068035 lies in the fact that, in order to create localised vacuum, debris may be also sucked together with air, which, if larger in size than the suction hole, may obstruct the same hole, thereby preventing the surrounding tissue from collapsing between the two gripping members and making the anchoring action of the device to the wall of the body cavity partially or wholly ineffective.

A further problem relates to the fact that, when the tissue, collapsing between the two gripping members reaches the suction hole, the latter is blocked, so that only a part of the surrounding tissue is effectively sucked in between the two gripping members. Consequently the tissue is partially gripped, which may be ineffective for the locomotion phase in that, during the phase of elongation or contraction of the tubular body of the endoscopic device, disengagement of the tissue may occur, with consequent loss of grip by the front end or the rear end of the device, thus creating an inefficient locomotion movement.

Moreover, when the tissue reaches the suction hole, obstruction of the hole is accompanied by the entry of a portion of tissue in said hole. Due to the long suction action lasting throughout the phase of elongation or contraction of the tubular body, and to its high intensity (-0.7 bar), the edge of the hole may damage the mucosa and some underlying tissue layers with consequent flare and, at times, bleeding.

US 2004/0143159 A1 discloses a self-propelling catheter introducing system with a propulsion device including gripper portions in the form of perforated suction rings placed at the ends of a steering/elongation portion. The gripping portions provide a suction action so that they can selectively attach to the inside surface of the body cavity whenever a vacuum is applied through the perforated rings.The perforated rings allow movement of the catheter by coordinated motion and control of the gripper portions and the steering elongation portion.

The object of the present invention is to provide an endoscopic device suitable for autonomous locomotion within a body cavity, for example according to patent application no. WO/02/068035, wherein the temporary and alternating anchorage of the front and rear ends of the device to the wall of the body cavity is stable and evenly distributed on the entire perimeter of said ends, such as to avoid obstruction of the suction hole.

Another object of the present invention is to provide an endoscopic device of the aforementioned type wherein anchorage of its ends to the walls of the body cavity takes place without risks of damage to the tissue involved in the action of anchorage.

These objects are achieved with the endoscopic device according to the present invention whose main features are set forth in claim 1.

The main feature of the endoscopic device according to the invention consists in that, on the multichannel support whereon the mobile member of the anchoring means slides and wherein the suction hole is formed, an evenly perforated coaxial diaphragm is placed in a spaced relationship from the surface of said multichannel support, the width of the perforations being smaller than that of the suction hole. In a preferred embodiment the diaphragm is formed by a hollow cylindrical member on whose lateral surface a plurality of longitudinal apertures are formed, with width smaller than that of said hole and evenly distributed on said surface. The cylindrical member therefore performs the triple function of filter, distributor of the suction effect and spacer separating the tissue from the suction hole.

Other features, and advantages, of the endoscopic device according to the present invention will be made clearer by the following description of one of its embodiments, given by way of a non-limiting example, in which:
- Figure 1 is a schematic overall view of the improved endoscopic device according to the present invention;

- Figure 2 is an enlarged longitudinal view of the front end portion of the endoscopic device of Figure 1;
- Figure 3 is a cross-sectional view of the device according to the invention taken along line III-III of Figure 2;
- Figure 4 is an enlarged longitudinal cross-sectional view of a front end portion of the endoscopic device according to the invention taken along line IV-IV of Figure 3;
- Figure 5 is a perspective view of a filtering body used in the endoscopic device according to the invention.

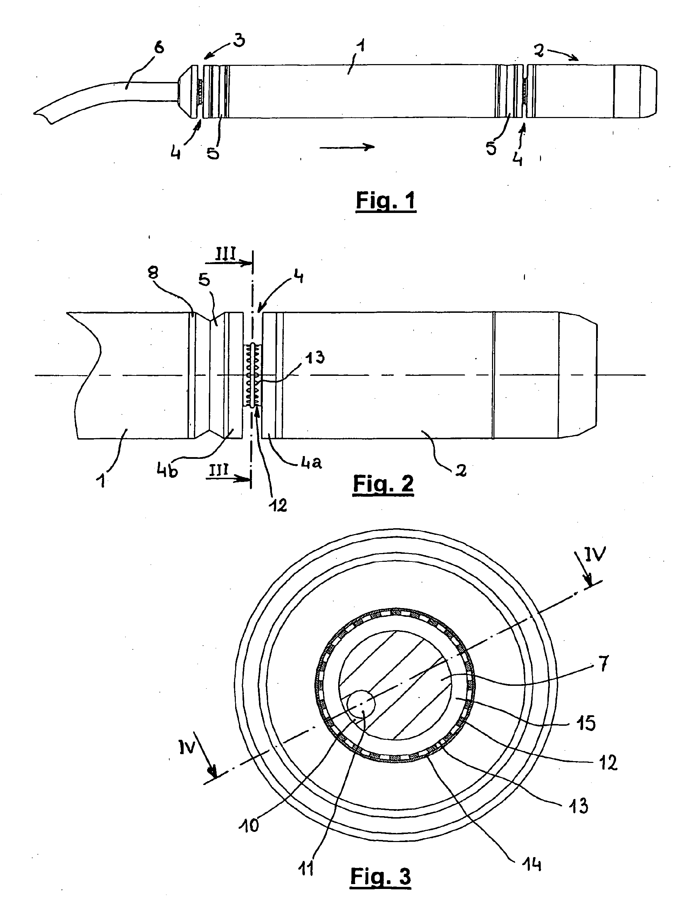

Referring to Figure 1, the endoscopic device according to the present invention comprises a tubular body 1 with variable length, extending between two end sections, called front end section 2 and rear end section 3 respectively, the front and rear ends being referred to the direction of forward locomotion of the device in the body cavity indicated by the arrow F. Clearly the device will be able to move forwards and backwards in the body cavity.

The front end 2 and rear end 3 comprise anchoring means 4, in particular of the clamp type, whereby the device attaches temporarily and alternately to the wall of the body cavity to allow, in a known manner, its locomotion. More particularly the movement of locomotion is achieved as a result of an alternation of elongations and contractions of the tubular body 1, achieved pneumatically, at which the rear end 3 or, respectively, the front end 2, are temporarily anchored to the body cavity via the respective anchoring means 4.

The elongations and contractions of the tubular body 1 are achieved by pressurising or, respectively, depressurising an internal chamber thereof. For this purpose the tubular body 1 may have a bellows configuration, as described for example in patent application WO/02/068035, or, preferably, may be made in an elastic material incorporating a reinforcement structure distributed over its length, substantially rigid in its radial direction and yielding in the axial direction, as described in European patent application no. EP 05425854 in the name of the same Applicant. More particularly the reinforcement structure consists of a spring 19, preferably a pair of coaxial springs with opposite direction of rotation of the relative coils, incorporated in a silicone tube 20 as described in the aforementioned European patent application.

The anchoring means 4 of the clamp type provided at the front end 2 and rear end 3 of the endoscopic device according to the invention are operated by respective pneumatic actuators 5 that can be made in the same way as the tubular body 1. The rear end 3 is connected to an external control unit by means of a tube 6 conveying the service tubes, including those for admitting compressed air to the tubular body 1 or for creating a vacuum therein, achieving in this way the elongation and the contraction of the tubular body 1 required for locomotion of the device.

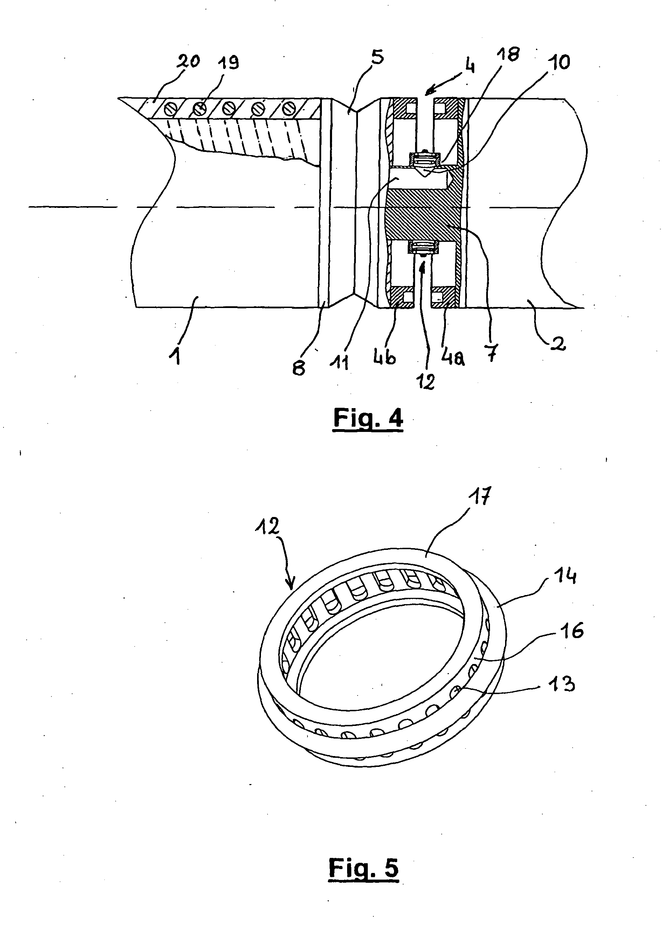

As illustrated in greater detail in Figures 2 and 3, relating to the front end 2 (the rear end 3 is equivalent), the clamp anchoring means 4 are formed by a pair of substantially circular jaws 4a and 4b, the first of which is fixed, while the second is mobile in relation to the first. In particular the mobile jaw 4b is mounted slidingly on a multichannel support 7, which can be seen in Figures 3 and 4, extending perpendicularly from a connection flange 8 axially connecting the end 2 to the tubular body 1. The multichannel support 7 is illustrated as solid in the drawings for reasons of simplicity, but actually it has several axial channels for the movements actuator fluid, washing of the TV camera, passage of electrical cables and for other accessory services.

The pneumatic actuator 5 of the clamp means 4, schematised as bellows 9 yet which can also be made in silicone material incorporating a helical spring as described in the aforementioned European patent application EP 05425854, is placed between the flange 8 and the jaw 4b. The pressurisation or the depressurisation of the actuator 5 corresponds to an elongation or a contraction of the bellows 9 or, alternatively, of the reinforced elastic pipe, which in turn corresponds to a sliding of the mobile jaw 4b in one direction or in the opposite one and the consequent closure or, respectively, opening of the anchoring means 4.

In the multichannel support 7, in a substantially intermediate position between the jaws 4a and 4b, a suction hole 10 is formed, communicating via a conduit 11 with an external suction unit not shown. At the suction hole 10 on the multichannel support 7 a tubular cylindrical body 12, or filter, is placed, having a plurality of longitudinal apertures 13, evenly distributed circumferentially on its lateral surface. At an intermediate position of the lateral surface of the filter 12 a perimetrical ribbing 14 is formed in a relatively soft material, for example Shore A50 silicone.

As shown in detail in Figures 4 and 5, the tubular cylindrical body 12, or filter, is formed by an annular diaphragm 16, on which the longitudinal apertures 13 are formed, and by two flanges 17 extending radially towards the interior of the diaphragm 16 from its perimetrical edges. The filter 12 is force fit mounted on the multichannel support 7, causing one of its radial flanges 17 to abut against a shoulder 18 to align it with the suction hole 10. The two radial flanges 17 also act as spacers between the diaphragm 16 and the surface of the tubular member 7 forming a chamber 15 between them.

Each longitudinal aperture 13 has a width smaller than half the diameter of the suction hole 10 of the conduit 11, so that any debris passing through the filter 12 does not block the suction conduit 11, while debris larger in size than the single aperture may obstruct the same aperture, although it is not detrimental to the suction action.

Thanks to the chamber 15 between the diaphragm 16 of the filter 12 and the multichannel support 7, the suction exerted through the suction hole 10 is distributed substantially evenly in the chamber 15 and, through the apertures 13 of the filter 12, outside of the filter. In this way, when the tissue of the body cavity, drawn between the two jaws 4a, 4b by suction, reaches one of the apertures 13, the suction action is not interrupted until all the apertures have been blocked by the tissue. This situation is the desirable one in that, in this way, the surrounding tissue collapses evenly between the two jaws, achieving an even grip necessary for an effective locomotion step.

The presence of the intermediate perimetrical ribbing 14 on the filter 12 prevents the tissue of the body cavity from collapsing in the apertures 13, even continuing the suction action, thereby eliminating or, in any case, minimising the risk of damage to the mucosa.

Variants and/or modification may be made to the improved endoscopic device according to the present invention, without departing from the scope of the invention, as set forth in the following claims.

## Claims

1. Endoscopic device suitable for moving autonomously in a body cavity in a prefixed direction of forward locomotion, comprising a tubular body (1) of variable length extending between a front end section (2) and a rear end (3) section comprising respective anchoring means (4) for fixing temporarily and alternately said ends to the wall of the body cavity in synchronism with corresponding axial elongations and contractions of said tubular body (1) consequent to the injection of compressed air and, respectively, creation of vacuum therein, and pneumatic actuator means (5) for actuation of said anchoring means (4), said anchoring means comprising a fixed jaw (4a) and a mobile jaw (4b) connected to said actuator means (5) and sliding axially on a multichannel support (7) wherein at least one suction hole is formed (10) between said two jaws (4a, 4b), so that between said two jaws a vacuum is generated for drawing between them the surrounding tissue of said body cavity that, in this way, can be temporarily gripped between the jaws of one and the other end alternately, **characterised in that**, coaxially to said multichannel support (7), an evenly perforated diaphragm (12) is positioned at said at least one suction hole (10), in a spaced relationship from the surface of said multichannel support (7), the width of the perforations being smaller than that of the suction hole.

2. Endoscopic device according to claim 1, wherein said diaphragm (12) is formed by a hollow cylindrical member (12) having lateral surface (16) on which a plurality of longitudinal apertures (13) are formed, the width of said apertures being smaller than that of said hole (10) and evenly distributed along said surface.

3. Endoscopic device according to claim 1 or 2, wherein on said cylindrical member (12) at least one perimetrical ribbing (14) is provided, extending in an intermediate position from said lateral surface.

4. Endoscopic device according to claim 3, wherein said perimetrical ribbing (14) is in a relatively soft material.

5. Endoscopic device according to anyone of claims 2, 3 and 4, wherein said cylindrical member (12) is formed by an annular diaphragm (16) and by two flanges (17) extending radially and inwardly from the perimetrical edges of said diaphragm.

6. Endoscopic device according to any one of the previous claims, wherein said cylindrical member (12) is force fit mounted on said multichannel support (7) and abuts against a shoulder (18) formed on the support.

7. Endoscopic device according to any one of the previous claims, wherein said longitudinal apertures (13) have a width smaller than half the diameter of said hole (10).

8. Endoscopic device according to any one of the previous claims, wherein said tubular body with variable length (1) is made in an elastic material incorporating a reinforcement structure (19) distributed over its length and substantially rigid in its radial direction while being yielding in its axial direction.

## Patentansprüche

1. Endoskopische Vorrichtung, die zum selbständigen Fortbewegen in einem Körperhohlraum in einer festgelegten Richtung einer Vorwärtsbewegung geeignet ist, enthaltend einen rohrartigen Körper (1) einer variablen Längsausdehnung zwischen einem Vorderendeabschnitt (2) und einem Hinterendeabschnitt (3), enthaltend jeweilige Ankereinrichtungen (4), um die Enden temporär an der Wand des Körperhohlraums in Synchronisation mit entsprechenden axialen Ausdehnungen und Kontraktionen des rohrartigen Körpers (1) als Folge der Injektion von Druckluft bzw. Erzeugen von Vakuum darin zu fixieren, und pneumatische Aktuatoreinrichtungen (5) zur Betätigung der Ankereinrichtungen (4), welche Ankereinrichtungen enthalten eine fixierte Backe (4a) und eine mobile Backe (4b), die mit den Aktuatoreinrichtungen (5) verbunden ist und axial an einem Mehrkanalsupport (7) gleitet, worin wenigstens ein Saugloch (10) zwischen den zwei Backen (4a, 4b) ausgebildet ist, so dass zwischen den zwei Backen ein Vakuum erzeugt wird, um das umgebende Gewebe des Körperhohlraums zwischen sie zu ziehen, das auf diese Weise abwechselnd zwischen den Backen des einen und des anderen Endes temporär gegriffen werden kann, **dadurch gekennzeichnet, dass** koaxial zu dem Mehrkanalsupport (7) ein gleichmäßig perforiertes Diaphragma (12) an dem wenigstens einen Saugloch (10) in einer beabstandeten Relation von der Oberfläche des Mehrkanalsupports (7) positioniert ist, wobei die Weite der Perforationen kleiner als jene des Sauglochs ist.

2. Endoskopische Vorrichtung nach Anspruch 1, wobei das Diaphragma (12) durch ein hohles zylindrisches Element (12) gebildet ist, das eine seitliche Oberfläche (16) hat, an welcher eine Mehrzahl von länglichen Öffnungen (13) ausgebildet ist, wobei die Weite der Öffnungen kleiner als jene des Lochs (10) und die gleichmäßig längs der Oberfläche verteilt sind.

3. Endoskopische Vorrichtung nach Anspruch 1 oder 2, wobei an dem zylindrischen Element (12) wenigstens eine perimetrische Riffelung (14) vorgesehen ist, die sich in einer Zwischenposition von der seitlichen Oberfläche erstreckt.

4. Endoskopische Vorrichtung nach Anspruch 3, wobei die perimetrische Riffelung (14) in einem relativ weichen Material ist.

5. Endoskopische Vorrichtung nach einem der Ansprüche 2, 3 oder 4, wobei das zylindrische Element (12) durch ein ringartiges Diaphragma (16) und durch zwei Flansche (17) gebildet ist, die sich radial und einwärts von den Umfangsrändern des Diaphragmas erstrecken.

6. Endoskopische Vorrichtung nach einem der vorhergehenden Ansprüche, wobei das zylindrische Element (12) an dem Mehrkanalsupport (7) durch Presspassung montiert ist und gegen eine Schulter (18) anliegt, die an dem Support ausgebildet ist.

7. Endoskopische Vorrichtung nach einem der vorhergehenden Ansprüche, wobei die länglichen Öffnungen (13) eine Weite haben, die kleiner als die Hälfte des Durchmessers des Lochs (10) ist.

8. Endoskopische Vorrichtung nach einem der vorhergehenden Ansprüche, wobei der rohrartige Körper (1) mit variabler Länge in einem elastischen Material hergestellt ist, das eine Verstärkungsstruktur (19) beinhaltet, die über seine Länge verteilt und im Wesentlichen steif in ihrer radialen Richtung ist, während sie in ihrer axialen Richtung nachgiebig ist.

## Revendications

1. Dispositif endoscopique apte à se déplacer de façon autonome dans une cavité d'un corps selon une direction prédéterminée de déplacement vers l'avant, comprenant un corps tubulaire (1) de longueur variable s'étendant entre une section d'extrémité avant (2) et une section d'extrémité arrière (3) comportant des moyens d'ancrage respectifs (4) pour fixer temporairement et alternativement lesdites extrémités à la paroi de la cavité du corps en synchronisme avec des allongements et des raccourcissements axiaux correspondants du dit corps tubulaire (1) suite respectivement, dans ce dernier, à l'injection d'air comprimé et à la création d'un vide, et des moyens actionneurs pneumatiques (5) pour actionner lesdits moyens d'ancrage (4), lesdits moyens d'ancrage comportant une mâchoire fixe (4a) et une mâchoire mobile (4b) reliées aux dits moyens actionneurs (5) et glissant axialement sur un support multi-canal (7) où au moins un orifice d'aspiration est formé (10) entre lesdites deux mâchoires (4a, 4b), de façon qu'un vide soit produit entre lesdites deux mâchoires pour attirer entre elles les tissus environnant de ladite cavité du corps qui, de cette façon, peuvent être temporairement saisis entre les mâchoires alternativement de l'une et de l'autre extrémité, **caractérisé en ce que**, de façon coaxiale au dit support multi-canal (7), un diaphragme (12) perforé de façon régulière est positionné sur ledit au moins un orifice d'aspiration (10) à une certaine distance de la surface du dit support multi-canal (7), la largeur des perforations étant plus petite que celle de l'orifice d'aspiration.

2. Dispositif endoscopique selon la revendication 1, où ledit diaphragme (12) est constitué d'un élément cylindrique creux (12) ayant une surface latérale (16) sur laquelle est réalisée une pluralité d'ouvertures longitudinales (13) régulièrement distribuées le long de ladite surface, la largeur des dites ouvertures étant plus petite que celle du dit orifice (10).

3. Dispositif endoscopique selon l'une des revendications 1 et 2, où au moins une nervure périphérique (14) est prévue sur ledit élément cylindrique (12), s'étendant dans une position intermédiaire à partir de ladite surface latérale.

4. Dispositif endoscopique selon la revendication 3, où la nervure périphérique (14) est en un matériau relativement souple.

5. Dispositif endoscopique selon l'une quelconque des revendications 2, 3 et 4, où ledit élément cylindrique (12) est constitué d'un diaphragme annulaire (16) et de deux brides (17) s'étendant radialement et vers l'intérieur à partir des bords périphériques du dit diaphragme.

6. Dispositif endoscopique selon l'une quelconque des revendications précédentes, où ledit élément cylindrique (12) est monté en force sur ledit support multi-canal (7) et abute contre un épaulement (18) réalisé sur le support.

7. Dispositif endoscopique selon l'une quelconque des revendications précédentes, où lesdites ouvertures longitudinales (13) ont une largeur plus petite que la moitié du diamètre du dit orifice (10).

8. Dispositif endoscopique selon l'une quelconque des revendications précédentes, où ledit corps tubulaire de longueur variable est réalisé dans en matériau élastique dans lequel est incorporée une structure de renfort (19) répartie sur sa longueur et relativement rigide dans sa direction radiale tout en étant flexible dans sa direction axiale.
